# EUROPEAN PATENT APPLICATION

(11) **EP 2 340 816 A1**
(43) Date of publication of application: **06.07.2011**
(21) Application number: 11150820.6
(22) Date of filing: 20.04.2009
(51) Int. Cl.: A61K 9/72, A61K 9/16

(54) **A plant for manufacturing composite carrier particles**

(30) Priority: 21.04.2008 PL 38498408
(62) Divisional of application: 09735696.8
(71) Applicant: Przedsiebiorstwo Produkcji, 51-131 Wroclaw (PL)
(72) Inventor: Gradon, Leon, 02-796, Warszawa (PL); Sosnowski, Tomasz, 01-917, Warszawa (PL); Pirozynski, Michal, 00-358, Warszawa (PL); Han, Stanislaw, 51-604, Wroclaw (PL)
(74) Representative: Krekora, Magdalena

(57) **Abstract**

The plant for manufacturing carrier particles is provided with a system (1) for generation of liquid aerosol from solution/suspension, followed by a multi-step drying system (2) with controlled heating zones, a system (3) for pre-separation of a non-respirable fraction of powder particles, and a respirable fraction separation system (4).

## Description

### TECHNICAL FIELD

The object of the present invention is a plant for manufacturing composite carrier particles in a form of a solid particle being a composite of surfactants and mucolytics (mucolytic substances), which is a carrier of a drug delivered into the organism by inhalation.

### BACKGROUND ART

Aerosol therapy is one of the most expansively developing techniques of drug administration. The respiratory system, which is the broadest way of contact between the organism and external environment, provides the opportunity for effective delivery of the drug in a system therapy. A biologically active ingredient, deposited in the area of alveoli, can be released to the circulatory system in a controlled way. Modem technical solutions offer drug particles containing biologically active ingredients acting as analgesics or antibiotics. Insulin may be administered by inhalation as well.

The aerosol therapy is a basic method of treatment of the respiratory system, including such diseases of civilization like asthma. It is also the main method of treatment for the chronic obstructive pulmonary disease (COPD). While getting into the respiratory system, the drug is conversed into the aerosol form, i.e. a stable dispersion of droplets of liquid or solid particles in the inhaled air. The drug in liquid form is conversed into the aerosol by atomization with ultrasonic waves, or by dispersing in nozzle with compressed air. The drug in solid form is pulverized and conversed into aerosol from a suspension in a volatile medium, which was at first produced under increased pressure, and then expanded in the nozzle. The volatile agent evaporates, and particles form aerosol which is inhaled by a patient. Due to lack of coordination between inspiration and the moment of aerosol release from a pressurized container as well as high aerosol jet rate, it is impossible that the medication flow is effectively oriented by the inspiration from the trachea. Thus the medication jet hits the back wall of the throat with all its energy, giving undesired drug deposition in that part of the respiratory system, and only slight portion of the medication penetrates to the bronchial tree. Therapeutic effect of inhalation depends on size of aerosol particles, thus on the initial size of powder particles. Correct result is obtained when the diameter of aerosol particle is below 5 µm. Present developments show improvements in aerosol therapy when powder particles that form the aerosol, have diameters of hundreds of nanometers. In order to achieve high rate of pulverization of the particles of active ingredient, they are deposited on a surface of bigger particles that are pharmacologically neutral. During inhalation, if the inhaler is of proper construction, particles of the active ingredient detach from the carrier. The carrier particles, having large diameters, are captured in head airways, while the smaller drug particles enter the further parts of the respiratory system. Having been deposited in the bronchial tree, the drug particles penetrate through a layer of mucus that covers bronchi, and enter the receptors located in cytoplasm of epithelium cells. Effectiveness of penetration through mucus is diminished due to its stickiness. A great portion of drug particles, prior to penetration through the mucus layer, is removed from the deposition area through effective mucus and ciliary transport. The therapeutic power is badly reduced in case of respiratory tract illnesses connected additionally with mucus overproduction (e.g. mucoviscidosis or chronic bronchitis) and increasing stickiness thereof. Because of a thick and compact mucus cover the drug cannot get under it and react with receptors. In order to reduce that negative effect, a combined therapy is applied where the drug is administered by inhalation, in combination with oral administration of mucolytics for thinning the mucus.

Recent investigations, where drugs were administered concurrently with introduction of mucolytics and surfactants on the mucus surface, have proved that in such a case a mucolytic agent brings about the mucus thinning, while phospholipids that are present in the surfactant, destroy the mucus structure and its cohesion. As a consequence the mucus layer breaks up, which promotes effective transport of drug particles to the bronchial epithelium layer. Such treatment is possible only in a hospital setting. Besides, it is limited to the trachea area and the first bifurcations of the bronchial tree.

A bioactive preparation in form of powder and a method of its manufacturing is known from a patent specification PL195212, which describes method of manufacturing of particles for administering bioactive substance into a patient's respiratory tract. The particles can be administered as dry powders or in form of a stabilized dispersion containing non-aqueous continuous phase. Particularly advantageous is to deliver the particles by using a dry powder inhaler, metered-dose inhaler or a nebulizer.

A pharmaceutical preparation containing insoluble active ingredient is known from a patent specification PL378270, describing a pharmaceutical preparation for intrapulmonary administration, in form of particles containing a fraction of active ingredient in a lipid matrix, where solubility of the active ingredient in water is below 1.0 mg/ml. In one of the forms, the diameter of at least 90% of active ingredient particles in the pharmaceutical preparation is below 3 µm. In another one - the insoluble active ingredient is amfotericin B. The diameter and shape of particles can be such as to easy deliver the pharmaceutical preparation in aerosol form deep into lungs.

From a patent specification PL354572 there are known solid pharmaceutical preparations containing nanosols that consist of at least one, at least partially charged active ingredient in nanomol form, where the active ingredient is combined with opposite-charged chitozan derivative, as well as a method of manufacturing thereof, and their use for manufacturing of medicinal products.

Pharmaceutical preparations and manufacturing thereof, known from a patent specification PL357808, include compositions containing active particles containing active ingredient manufactured by following steps: providing of emulsion where dispersed phase contains solution of active ingredient in a solvent, and inducing formation of solid particles containing active ingredient in the emulsion. The particles can be separated from the emulsion. The invention also refers to active particles having standardized kurtosis not less than 5 and median diameter less than 100 µm.

Powder for application in a dry powder inhaler, composition of a carrier particle and a method of such carrier manufacturing are known from a patent specification PL196951. The powder for use in a dry powder inhaler consists of an active ingredient and a carrier, where lubricant percentage in the carrier ranges from 0.05 to 0.5% w/w, and carrier particles are at least partially coated with lubricant particles.

Attempts to bind surfactant with drug particles are known from a patent specification US5855913. This specification discloses porous particles of biodegradable polymer having apparent density below 0.4 g/cm³, containing drug and surfactant adsorbed on its surface, whose purpose is to facilitate resuspension of such particles from the powder structures.

Composite of such type does not meet however the requirements and does not show the properties mentioned above, that are the object of the present invention.

### DISCLOSURE OF INVENTION

The composite carrier for powdered drugs manufactured in the plant being the object to the present invention consists in that the composite carrier is in form of particles permanently bound into aggregates containing mucolytic substances and surfactants in the structure. Preferably, the carrier particle is microporous, with particularly developed surface defects. Preferably, the active ingredient, permanently bound by carrier particles, has the aerodynamic diameter smaller than 5 µm. Preferably, the mucolytic substance incorporated in the particle structure is in liquid or solid form under standard conditions at the temperature of 25°C and under pressure of 1 atm. Preferably, mucolytic substances are contained in the carrier particle structure, and are incorporated into the carrier structure in form of solid phase, preferably crystalline; and furthermore, the specific volume of the mucolytic substance is different from the specific volume of the carrier material. Preferably, surfactant is introduced into a volume structure of the carrier composite and also adsorbed on the surface of that structure. Surfactant is introduced into a volume solution of the carrier composite in the solvent and then adsorbed on the surface of the interface between solution and surrounding gas phase when the droplet of the solution is formed. Preferably, quantities of the mucolytic substance and the surfactant that are introduced into the carrier particle structure are sufficient to activate the drug transport process within a deposition area covering at least 1 mm² of mucus surface. The carrier particle structure diameter may be below 3.5 µm. Preferably all constituents of the drug composite carrier are water-soluble. Preferably the carrier particle contains lactose. Preferably mannitol is used as a mucolytic substance. Preferably dipalmitoylphosphatidylcholine (DPPC) is used as a surfactant.

The drug carrier manufacturing method utilized in the plant being the object of the invention consists in that the composite carrier particles are manufactured from a solution of the composite constituents, by atomization of the solution to the form of droplets, followed by controlled evaporation of the solvent, and segregation of dried particles. Preferably, the solution of substances that are the composite constituents is a solution of a main substance, mucolytic substance and surfactant in a volatile solvent, where such solvent is common for all dissolved constituents. Preferably, the solution is atomized to droplets of initial diameters not greater than 30 µm. Preferably, droplets of solution are formed by pneumatic or ultrasonic method. Preferably, solvent evaporates whilst droplets pass through a multi-step drying system preset for a particular temperature profile. Preferably, solvent evaporates in a multi-step drying system, with controlled heating zones, and preset for a particular temperature profile, in a spray-drying process, where the temperature at the inlet to the system is at least 10°C higher than the solvent boiling point, and the temperature in exit section of the drying system is at least 10°C lower than the solvent boiling point. Preferably, carrier particles during the spray-drying process acquire the carrier particles of expanded surface structure due to microcracks of the solid phase are formed. Preferably, the dried particles on leaving the air flow drying system enter a pre-separation system, where non-respirable fraction of powder particles greater in size than 5 µm is separated; then the remaining fraction of particles smaller than 5 µm is separated in a respirable fraction separation system, and the separated powder is received from a collector as a final product. Preferably, dried particles of the composite carrier are obtained from a water solution containing lactose as a main component, mannitol as mucolytic substance, and dipalmitoylphosphatidylcholine (DPPC) as surfactant.

The plant according to the invention consists in that the plant is provided with a system for generation of a liquid aerosol from a multicomponent solution/suspension, then a multi step drying system with controlled heating zones, and after that a system for pre-separation of non-respirable fraction of powder particles.

Preferably aerosol generation system is a pneumatic atomization system.

Preferably the aerosol generation system is a ultrasonic generation system.

Preferably the non-respirable powder fraction pre-separation system is an inertial dust collector.

Preferably the respirable fraction separation system is a high-performance dedusting system for extraction of particles of aerodynamic diameter below 5 µm.

The composite carrier for powdery drugs according to the invention is distinguished by the particle diameter that corresponds to the size of particles from the respirable fraction, delivered to the respiratory system by inhalation. The composite drug carrier improves drug penetration through a mucus layer once the particle has been deposited on the surface of the respiratory tract. This diameter may be adjusted by selecting the concentration of active substances in the water solution from which the primary droplets are generated, and then in the process of drying solid particles. The particle diameter may be also adjusted by selecting a method of the solution atomization. By adoption of a suitable temperature profile in the drying system allows for obtaining of particles of microporous structure, what results in apparent density of the composite material below 0,8 g/cm³. Moreover, the surface structure of particles is adjusted be by selection of the solution composition and the temperature profile in the drying process. As a result particles showing low tendency to aggregate are obtained what makes their aerosolization easier.

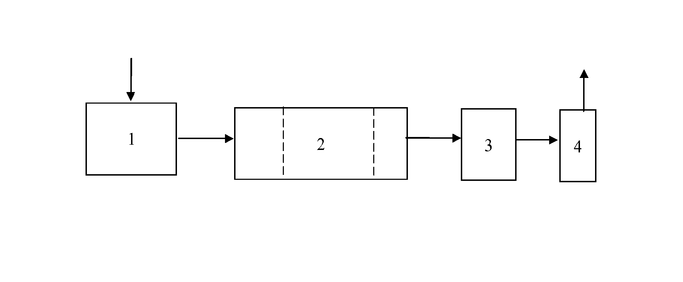

### BRIEF DESCRIPTION OF THE DRAWING

The plant for manufacturing composite carrier particles is presented at the drawing, where 1 is a system for liquid aerosol generation from a multicomponent solution/suspension, 2 is a multi-step drying system with controlled heating zones, 3 is a system for preseparation of a non-respirable fraction of powder particles, and 4 is a respirable fraction separation system.

### BEST MODE OF CARRYING OUT THE INVENTION

The subject of the inventions is presented in the following examples below, however these examples do not limit the scope of these inventions.

### Example 1

A composite carrier for powdery drugs contains the active ingredient administered by inhalation in form of particles permanently bound in aggregates with particles of the composite carrier containing mucolytics (mucolytic substances) and surfactants in its structure. The carrier particle is microporous, with particularly developed surface defects, its aerodynamic diameter is less than 5 µm. Mucolytic substance, embedded in the particle structure is in liquid state under standard conditions at the temperature of 25°C and under pressure of 1 atm, whereas surfactant is introduced into a volume structure of the carrier composite and also adsorbed on the surface of that structure during the process of spray drying. Moreover, quantities of the mucolytic substance and the surfactant that are introduced into the carrier particle structure, are sufficient to activate the drug transport process within a deposition area covering at least 1 mm² of mucus surface, while all constituents of the drug composite carrier are water-soluble.

### Example 2

A composite carrier for powdery drugs, prepared as described under Example 1, except that the carrier particle contains mucolytic substances in its structure; the mucolytic substances are embedded in the structure, and under standard conditions at the temperature of 25°C and under pressure of 1 atm they occur in form of solid, crystalline phase, and furthermore, the specific volume of the mucolytic substance is different from the specific volume of the carrier material.

### Example 3

A composite carrier for powdery drugs, prepared as described under Example 1, except that the carrier particle containing lactose, has in its structure mucolytic substances in form of non-ionic osmotic agent mannitol, and surfactant which is a chemical compound dipalmitoylphosphatidylcholine (DPPC). Analytical data resulting from tests and research show that a single composite particle releases the surfactant contained therein at amount of 1.17·10⁻⁴ mg/mm³, and surface tension of pure water decreases from the initial value of 72 mN/m to the value of 44.1 mN/m when the water surface became saturated with a monolayer of surfactant. Such effect indicates that the surfactant contained in composite shows a strong surface action. Apparently, on dissolving the composite particle, the surface activity of surfactant combined with lactose is greater than the activity of a pure surfactant. In addition, changes produced by the action of mannitol from the new composite particle, in viscosity of 0.1% mucin solution were measured, the mucin solution was assumed as bronchial mucus reference standard. It follows from the obtained results that mannitol in quantity of 0.05 mg per 1 g of the mucus standard reduces its viscosity more than three times.

The above results prove that a composite particle, containing a carrier in form of lactose, with added surfactant (DPPC) and mucolytic substance (mannitol), obtained by the new method, has a diameter corresponding to the respirable fraction - in terms of size of particles delivered into the respiratory system by inhalation. The diameter may be adjusted by selection of concentrations of active ingredients in the water solution, from which the primary droplets are generated, and then in the drying process of the solid particle. By applying suitable temperature profile in the drying system, the obtained particles may get microporous structure, and the apparent density of the composite material is smaller than 0.8 g/cm³. Furthermore, composite material obtained in such a way shows advantageous properties: it liquefies mucus and is surface-active, what activates transport of a drug particle attached to the carrier particle under the mucus surface and thus intensifies therapeutic effects.

### Example 4

The drug carrier manufacturing method consists in that the composite carrier particles are obtained from a solution containing a main substance, mucolytic and surfactant in volatile solvent, common for all dissolved constituents, in the process of the solution atomization to the droplets of diameters initially not greater than 30 µm and the said droplets are formed from the solution by pneumatic method in the liquid aerosol generation system 1. Solvent evaporates whilst droplets pass through the multi step drying system 2 , with controlled heating zones, and preset for a particular temperature profile, in a spray-drying process, where the temperature at the inlet to the oven is at least 10°C higher than the solvent boiling point, and the temperature in the exit zone of the oven is at least 10°C lower than the solvent boiling point. On leaving the flow type oven, the dried particles are separated in the non-respirable fraction pre-separation system 3, where a fraction of powder particles greater in size than 5 µm is separated; and the remaining fraction of particles smaller than 5 µm, having passed through the respirable fraction separation system 4, is received from a collector as a final product.

### Example 5

The drug carrier manufacturing is carried out as described in Example 4, except that the droplets of solution are formed in the liquid aerosol generation system 1 by ultrasonic method, and that the carrier particles formed during the spray-drying process in the multi step drying system 2 and provided with controlled heating zones, are characterized by expanded surface structure, due to microcracks of the solid phase.

### Example 6

The drug carrier manufacturing is carried out as described in Example 4 or Example 5, except that the drug carrier particles are obtained from a water solution containing lactose as a main component, at amount of 0.07 g per 1 ml of water, non-ionic osmotic agent mannitol as mucolytic, at amount of 0.06 g per 1 ml of water, to reduce mucus viscosity, and dipalmitoylphosphatidylcholine (DPPC) as surfactant, at amount of 0.05 mg per 1 ml of water. The solution is atomized to droplets in the liquid aerosol generation system 1, which is an ultrasonic generator of vibration frequency of 2.5 MHz, then the droplets are dried in the multi step drying system 2, which is a pipe oven with temperatures preset downstream to: 150°C, 100°C and 80°C, respectively. Particles of an average mass diameter below 3.5 µm are obtained in a respirable fraction separation system 4. Microscopic observations proved that the particles have a porous, morphologically developed microstructure, of apparent density 0.8 g/cm³.

### Example 7

A plant for manufacturing composite carrier particles comprises: a system 1 for liquid aerosol generation from a multicomponent solution/suspension, followed by a multi step drying system 2 with controlled heating zones, a system 3 for pre-separation of a non-respirable fraction of powder particles, and a respirable fraction separation system 4.

In this arrangement, a liquid aerosol in form of a mist composed of droplets suspended in air is produced from a multicomponent solution or suspension of carrier and active substances in a suitable solvent, in a system of aerosol generation 1, operating on a pneumatic, ultrasonic or other principle of atomization. A jet of fresh, cleaned and dried air flows in the generation system 1. The generated mist enters the drying system 2, where heating zones of different temperatures allow for precise control of the crystallization process that proceeds as a result of the solvent evaporation from the droplets. The obtained dry powder aerosol leaves the drying system 2, and undergoes a separation - first in the non-respirable fraction pre-separation system 3, and then - in the respirable fraction separation system 4. The pre-separator 3 can be an inertial dust collector, used for separation of particles having diameters out of the respirable limits, i.e. greater than 5 µm, whereas the respirable fraction separation system 4 is a high-performance dedusting system, for separation of particles smaller than 5 µm, that are a desired product used as a powder for inhalation. After the powder separation, the air jet flows out from the respirable fraction separation system 4, to the outside of the plant.

### Test results

Particles of the composite obtained according to the Example 1 and 3, having the average aerodynamic diameter of 3.5 µm, were placed in aerosolisation chamber and the effectiveness of their entrainment by the air flow of intensity 15 l/min was tested. The linear speed of the air in the chamber was 3.5 m/s. The effectiveness of the particles entrainment of the sample of powder exposed for 4 seconds amounted to:
For particles of pure lactose - 12%
For particles of the composite carrier - 42%

The enhanced effectiveness of the composite carrier in comparison to the lactose particles is connected with the fact that molecules DPPC associated with lactose are in their surface area oriented with the hydroxycarbon chains to the outside of the particle. As the result we can observe hindering effect. The particles of the composite carrier do not stick to each other and may be easily entrained. These effect does not occur in relation to the porous particles known from the prior art.

## Claims

1. A plant for manufacturing composite carrier particles, **characterized in that** the said plant is provided with a system for generation of a liquid aerosol (1) from a multicomponent solution/suspension, which system is followed by: a multi step drying system (2), with controlled heating zones; a system for pre-separation of non-respirable fraction of powder particles (3), and a respirable fraction separation system (4).

2. A plant according to claim 1, **characterized in that** the aerosol generation system (1) is a pneumatic atomization system.

3. A plant according to claim 1, **characterized in that** the aerosol generation system (1) is a ultrasonic generation system.

4. A plant according to claim 1, **characterized in that** the non-respirable powder fraction pre-separation system (3) is an inertial dust collector.

5. A plant according to claim 1, **characterized in that** the respirable fraction separation system (4) is a high-performance dedusting system for extraction of particles of aerodynamic diameter below 5 µm.
